# EUROPEAN PATENT APPLICATION

(11) **EP 3 929 587 A1**
(43) Date of publication of application: **29.12.2021**
(21) Application number: 20182274.9
(22) Date of filing: 25.06.2020
(51) Int. Cl.: G01N 33/68

(54) **CROSSLINKING REAGENT FOR BIOCONJUGATION FOR USE IN CROSSLINKING PROTEOMICS, IN PARTICULAR CROSSLINKING MASS SPECTROMETRY ANALYSIS**

(71) Applicant: Technische Universität Berlin, 10623 Berlin (DE)
(72) Inventor: RAPPSILBER, Juri, 10405 Berlin (DE); BELSOM, Adam, 10405 Berlin (DE); PEREZ-LOPEZ, Ana, 10115 Berlin (DE); SINN, Ludwig, 10829 Berlin (DE)
(74) Representative: Maikowski & Ninnemann Patentanwälte Partnerschaft mbB

(57) **Abstract**

The present invention relates to a crosslinking reagent for bioconjugation for use in crosslinking proteomics, in particular crosslinking mass spectrometry analysis, a method for obtaining the same and the use thereof for enriching peptide pairs.

## Description

The present invention relates to a crosslinking reagent for bioconjugation for use in crosslinking proteomics, in particular crosslinking mass spectrometry analysis, a method for obtaining the same and the use thereof for enriching peptide pairs.

### Description

Bioconjugation via the streptavidin-biotin system is thoroughly established as a universal tool in most fields of the biological sciences, including (but not limited to): Affinity chromatography, isolation studies, immobilising agents, selective elimination, flow cytometry, affinity cytochemistry, localisation studies, light microscopy, fluorescent microscopy, electron microscopy, histochemistry, signal amplification, immunoassay, diagnostics, gene probes, bioaffinity studies, epitope mapping, blotting technology, cytological probes, pathological probes, affinity therapy, drug delivery, fusogenic agents, affinity perturbation, affinity targeting, protein purification and crosslinking.

The streptavidin-biotin system is so widely used, partly because of the exceptionally high affinity, specificity and stability of the streptavidin-biotin complex. The streptavidin-biotin interaction is the strongest non-covalent biological interaction known (Kd ∼ 10⁻¹⁴ M). However, the strength of the interaction becomes a liability when reversal of the interaction is desired.

Methods attempting to reverse the interaction include extreme denaturing conditions, such as boiling in 6 M guanidinium hydrochloride at pH 1.5. This frustrates the range and scope of its application, where only conjugation is realistically feasible when dealing with sensitive study subjects. Extreme elution conditions can destroy the biological sample of interest and/or replace the undesired sample background with an equally deleterious background deriving from the enrichment procedure. Other problems associated with the use of biotin are its relative hydrophobicity and resultant insolubility, as well as its tendency to oxidise in certain preparative conditions (a particular problem for methods relating to mass spectrometry) (Villamor, J. G. et al. Profiling protein kinases and other ATP binding proteins in Arabidopsis using Acyl-ATP probes. Mol. Cell. Proteomics 12, 2481-2496 (2013)).

Crosslinking mass spectrometry analysis is a field of structural biology that could benefit enormously from the implementation of a truly reversible streptavidin-biotin based enrichment system. Crosslinking mass spectrometry involves the translation of temporal spatial proximity between and within proteins into covalent bonds. Proximal amino acid residues are crosslinked and proteins subsequently digested into complex mixtures of crosslinked and non-crosslinked peptides and peptide pairs, using enzymes. The resulting complex mixture is analysed by mass spectrometry and matched by database search, thus elucidating protein-protein interactions and revealing protein structure. Importantly, one of the largest advantages of crosslinking mass spectrometry technology is that it allows the study of proteins inside their native environment, the cell. A significant challenge to the approach, however, is that crosslinked peptide pairs containing the three-dimensional structural information following crosslinking, are typically of very low abundance, whereas analysis is usually performed on a much greater abundant modified and non-modified linear peptide mixture. Analysis is therefore akin to trying to find the proverbial needle in a haystack.

Currently, standard crosslinking mass spectrometry can routinely generate data on individual proteins and small-to-medium sized complexes, but when sample complexity increases beyond this, enrichment of crosslinked peptide pairs becomes a fundamental necessity, especially when scaling crosslinking upwards to cellular lysates, organelles and even living cells.

Crosslinking can be achieved using exogenous chemical crosslinking reagents, which represent a foundational pillar of crosslinking mass spectrometry. Such crosslinking reagents typically constitute two protein-reactive groups separated by a spacer (the simplest being an alkyl chain of varying length). The spacer can act as a passive distance restraint or can have additional functionality such as reactive or affinity handles grafted onto the crosslinker scaffold, which allow the capability for enrichment of crosslinked peptide pairs following enzymatic digestion of crosslinked protein. Any reactive or affinity handles present on a crosslinker need to be chemically inert, or at least non-reactive with the protein-reactive groups. In addition, crosslinkers should be chemically inert under the electrospray and gas-phase conditions of the mass spectrometer, unless there is the intention of generating specific reporter ions for the purposes of enhanced search strategies.

Analyte enrichment can be achieved in a three-step process. Firstly, the reactive or affinity group grafted on the crosslinker spacer must bind the crosslinked peptide pairs to a solid support. Secondly, the solid-support is washed to remove non-binding, non-crosslinker-modified linear peptides. Thirdly, solid-support bound crosslinked peptides are eluted prior to their analysis by mass spectrometry. For successful enrichment, all three steps must be completed. Crosslinking is one aspect of the broader field of bioconjugation.

In applications where solid-supported streptavidin is used to capture biotinylated moieties, the non-reversibility issue of the streptavidin-biotin system has led to numerous attempts at workarounds, including (but not limited to): using enhanced denaturing conditions, on-bead digestion of bound proteins or by implementing cleavable chemistry for biotin derivatives. Enhanced denaturing conditions can be characterised as either involving: 1. Attempts at competitive elution (Cheah, J. S. & Yamada, S. A simple elution strategy for biotinylated proteins bound to streptavidin conjugated beads using excess biotin and heat. Biochem. Biophys. Res. Commun. 493, 1522-1527 (2017)) or, 2. Attempts to reduce the binding affinity of one part of the system, either the "solid-support" component (with modified avidin molecules) (Vermette, P. et al. Immobilisation and surface characterisation of NeutrAvidin biotin-binding protein on different hydrogel interlayers. J. Colloid Interface Sei. 259, 13-26 (2003)) or the "tag" component (with reduced-affinity biotin analogs) (Garret-Flaudy, F. & Freitag, R. Use of the avidin (imino)biotin system as a general approach to affinity precipitation. Biotechnol. Bioeng. 71, 223-234 (2000)). None of these workarounds are fully satisfying when applied.

Competitive elution is an accepted concept and is already applied whereby both biotin and desthiobiotin are used as an eluent. Where excess biotin is currently used for attempting competitive elution of biotinylated bound derivatives, excess heating is also required. In the case of the Strep-tag^{®} system, competitive elution from the affinity column is achieved without extreme heating, using either biotin or the more weakly binding desthiobiotin. The Strep-tag^{®} system is used to purify proteins on a specially engineered solid-supported streptavidin (Strep-Tactin). Strep-Tag II is an 8 amino acid synthetic peptide that is N- or C-terminally fused to recombinant proteins and has high-specificity for Strep-Tactin, but a binding affinity lower than both desthiobiotin and biotin. DSB-X biotin is a desthiobiotinylated succinimidyl ester reagent that can either be used to reversibly bind tagged moieties to a solid support, or modify the solid-support itself for reversible binding of free protein (Hirsch, J. D. et al. Easily reversible desthiobiotin binding to streptavidin, avidin, and other biotin-binding proteins: uses for protein labeling, detection, and isolation. Anal.Biochem. 308, 343-357 (2002)). Competitive elution is used to reverse binding on desthiobiotin incorporated into chemical probes (Desthiobiotin-ATP), for studying nucleotide-binding proteins and grafted to DNA/RNA for the analysis of chromatin composition. In these cases, desthiobiotin is used as an affinity group to link two entities, where reversibility is required at one end.

Both biotin- and non-biotin-based enrichable crosslinking reagents have been developed for crosslinking mass spectrometry. An enrichable crosslinker, using a biotin handle as an affinity group for crosslinking enrichment, even pre-dates the time when crosslinking was routinely being analysed by mass spectrometry (Hatanaka, Y., Hashimoto, M. & Kanaoka, Y. A novel biotinylated heterobifunctional cross-linking reagent bearing an aromatic diazirine. Bioorg. Med. Chem. 2, 1367-1373 (1994)). Since then, multiple biotin-exploiting crosslinkers have followed, whereby biotin is either directly grafted onto a crosslinker spacer region (Fujii, N., Jacobsen, R. B., Wood, N. L., Schoeniger, J. S. & Guy, R. K. A novel protein crosslinking reagent for the determination of moderate resolution protein structures by mass spectrometry (MS3-D). Bioorg. Med. Chem. Lett. 14, 427-429 (2004)), or subsequently added through a bioorthogonal transformation of another crosslinker functionality following crosslinking (Tan, D. et al. Trifunctional cross-linker for mapping protein-protein interaction networks and comparing protein conformational states. Elite 5, e12509 (2016)). Most recently there has been an attempt to move away altogether from a biotin-streptavidin based enrichment system for crosslink enrichment, with the introduction of an IMAC-enrichable phosphonic acid based crosslinking reagent (Steigenberger, B., Pieters, R. J., Heck, A. J. R. & Scheltema, R. A. PhoX: An IMAC-Enrichable Cross-Linking Reagent. ACS Cent Sei 5, 1514-1522 (2019)). Vanishingly few of these crosslinkers have progressed past the proof-of-principle stage, however.

The object of the present invention was to provide a tool that allows for an improved analysis of proteins by mass spectroscopy.

According to the invention this object is being solved by a reagent as described in the claims.

Accordingly, a crosslinking reagent for bioconjugation for use in crosslinking proteomics, in particular crosslinking mass spectrometry analysis, is provided, which comprises the general structure of general formula (I)

R¹-(CH₂)ₐ-(Y-CH₂)ₙ-XR²-(CH₂-Y)ₙ-(CH₂)ₐ-R¹

Wherein
- R¹ is selected from a functional group comprising ester, halogen, aldehyde, isothiocyanate, isocyanates, anhydride, epoxides, acetyl, glyoxal, triazine, hydrazine, disulfide, azide, ketone, phosphine, alkene, amines, N-heterocycle,
- a = 1-10, preferably 2-5, more preferably 2-3;
- Y is selected from -O-, -S-, -S-S-, -S(=O)-, -¹³CH₂-, -CD₂-, -¹⁸O-m
- n = 0-4, preferably 0, 1 or 2,
- X is selected from N, P, -CH-, aliphatic cyclic structures and aromatic cyclic structures, preferably N;
- R² comprises a moiety, in particular a biotin derivative which has a lower affinity to avidin or streptavidin than biotin.

Thus, the crosslinking agent according to the invention is a trifunctional agent which comprises at least two functional moieties as terminal moieties and a biotin derivative that is able to bind to streptavidin. The two terminal moieties (R¹) placed on the crosslinking agent can be identical (homobifunctional crosslinkers) or different (heterobifunctional crosslinkers).

The moiety R¹ may be selected from a group comprising N-hydroxysuccinimide esters, imidoesters, isothiocyanates, isocyanates, acyl chlorides, sulfonyl chlorides, aryl sulfonyl fluorides, acyl azides, fluorophenyl esters, anhydrides, fluorobenzene, epoxides, alpha,beta-unsaturated aldehydes, 1,3-ketoaldehydes, 1,2,3-triazines, 1,2-cyclohexanedione, 2-methoxy-3-oxindoles, phenylglyoxal, a-keto-oximes, 2-fluoro-5-nitrotropolone, O-phthalaldehyde, maleimides, halo acetyls, pyridyl disulfides, aryl azides, diazirines, benzophenones, psoralens, phoshines, alkenes, cyclooctynes, tetrazines, hydrazines, alkoxyamines-,

In an embodiment the terminal moiety R¹ may be ester groups such N-hydroxysuccinimide esters (NHS), imidoesters, isothiocyanates and isocyanates, but also may be acyl chlorides, sulfonyl chlorides, aryl sulfonyl fluorides, acyl azides, anhydrides, fluorobenzene, epoxides, aldehydes, 1,2,3-triazines, 1,2-cyclohexanedione, 2-methoxy-3-oxindoles, phenylglyoxal, a-keto-oximes and 2-fluoro-5-nitrotropolone. These terminal moieties are able to undergo a reaction with amino groups (primary amines -NH₂ or secondary amines -NH-) or hydroxyl groups (-OH), in particular on the side chain of Lys, Arg, His, Ser, Thr or Tyr in proteins.

Terminal moieties R¹ such as maleimides, halo acetyls and pyridyl disulfides are able to undergo a reaction with sulfhydryl groups (-SH) of Cys in proteins. Photoreactive terminal moieties such as aryl azides, diazirines, benzophenones and psoralens can react unspecifically with amino acid residues within proteins or with nucleic acids, such as DNA/RNA. The aldehyde/ketone groups present on other biomolecules (i.e. oxidised sugars of glycoproteins) can react with hydrazines and alkoxyamines when they are used as reactive sites of a crosslinking agent.

A different approach is the incorporation of noncanonical amino acids that enable site-specific or residue-selective labeling of proteins with alkyl azides, alkenes or trans-cyclooctenes. These bioorthogonal chemical groups allow covalent binding through a crosslinking agent by (a) Staudinger ligation between alkyl azides and phosphine reagents, (b) Cu(I)-catalysed cycloaddition between alkyl azides and alkynes to afford triazole adducts, (c) Cu-free cycloaddition between alkyl azides and activated cyclooctynes, or (d) tetrazine moieties can undergo selective and rapid Diels-Alder reactions with activated alkenes such as *trans-*cyclooctenes.

In a preferred embodiment said terminal moiety R¹ may be ester groups such as succinimide ester (NHS), phthalic-di-aldehyde; diazirine

The (at least) two functional terminal moieties are separated by a spacer comprising -(CH₂)ₐ-X-(CH₂)ₐ-. The spacer arm of the general structure (I) can be varied by its length (∼7-30 Å, a = 1-10, preferably 2-5, more preferably 2-3). The length of the spacer arm impacts the distance restraint information obtained from crosslinking analysis. Longer spacer arms are able to capture more distance restraints in proteins, but the information can be less informative for structural modelling than that derived from the use of shorter spacer arms.

The spacer arm can also be varied by its composition, which can affect subsequent hydrophobicity/hydrophilicity (i.e. polyethylene glycol chains increase hydrophilicity) or enhance data analysis capabilities through additional functionality (such as isotopically labeled chains for crosslinking quantitation and cleavable groups such as disulfides or sulfoxides).

The present crosslinking agent may be of an asymmetrical structure (II) 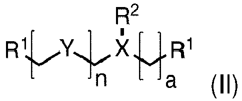 or a symmetrical structure (III) 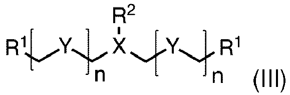 Wherein
- a = 1-10, preferably 2-5, more preferably 2-3.
- n = 1-4, preferably 1-2, more preferably 1.
- Y is selected from -O-, -S-, -S-S-, -S(=O)-, -¹³CH₂- , -CD₂-, -¹⁸O-.

The biotin moiety enables the specific interaction with a solid support comprising avidin or streptavidin for enriching crosslinked peptide pairs as described in more detail further below.

In an embodiment of the present crosslinking agent moiety R² comprises an alkyne or a biotin derivative wherein the cyclic moiety of biotin is different from that of biotin such as desthiobiotin, 2-iminobiotin, 3,4-diaminobiotin or chemically modified derivatives thereof such as carbonates or cabamates. It is mostly preferred if R² comprises desthiobiotin.

In a further embodiment moiety R² may be one of the following Biotin analogues (IV) or Desthiobiotin analogues (V): 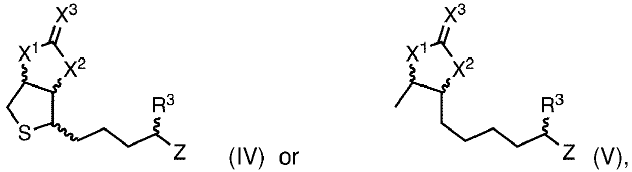 wherein
- X¹, X², X³ is selected from -NH₂, -NH-, -N-Me, -N-Et, -O-.
- R³ is selected from the group consisting of H, optionally substituted C₁-C₁₀ alkyl, optionally substituted C₃-C₁₀ cycloalkyl, optionally substituted C₂-C₁₀ alkenyl, optionally substituted C₃-C₁₀ cycloalkenyl, optionally substituted C₂-C₁₀ alkynyl, optionally substituted C₂-C₁₀ heteroalkyl, optionally substituted C₃-C₁₀ heterocycloalkyl, optionally substituted C₂-C₁₀ heteroalkenyl, optionally substituted C₃-C₁₀ heterocycloalkenyl, optionally substituted C₂-C₁₀ heteroalkynyl, optionally substituted C₆-C₁₄ aryl, optionally substituted C₅-C₁₄ heteroaryl;
- Z is selected from -COOH, halide aryl, preferably C₅-C₆ such as C₆-C₁₄ aryl, C₅-C₁₄ heteroaryl, alkyl halides, preferably C₁-C₄ halide, alkyl azides, preferably C₁-C4 azides, alkynes, preferably C₂-C₄ alkynes, alkenes, preferably C₂-C₄ alkenes, alkyl boronates, preferably C₁-C₄ boronate, aryl boronates, preferably C₅-C₆ boronates such as C₆-C₁₄ aryl boronates, C₅-C₁₄ heteroaryl boronates, alkyl tetrazines, preferably C₁-C₄ tetrazines, optionally substituted -CO-NH-(CH₂)_{b}-COOH wherein b = 1-10, preferably 2-6, optionally substituted -CO-NH-(CH₂-O-)_{b}-(CH₂)_{b}-COOH wherein b = 1-10, preferably 2-6.

All the stereocenters are selected for both configurations *S* and *R* (4-16 stereoisomers in total), preferably configuration *3S, 4S, 6R* for biotin analogues and configuration *3S, 4S* for desthiobiotin analogues.

Optionally, substituents in the valeryl side chain (R³) affects by decreasing the affinity towards Streptavidin, and therefore more suitable for reversible binding.

The crosslinking agent may have one of the following structures:

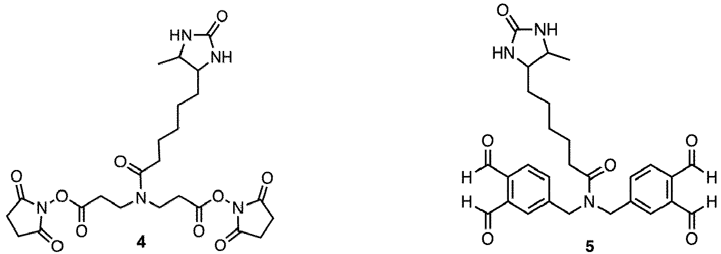

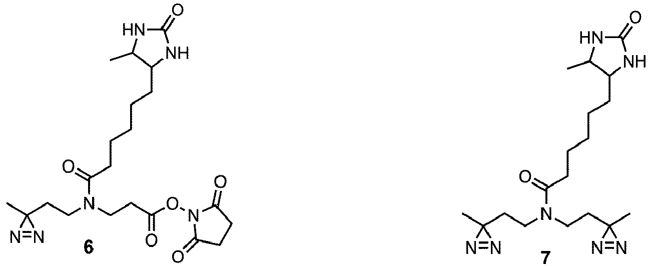

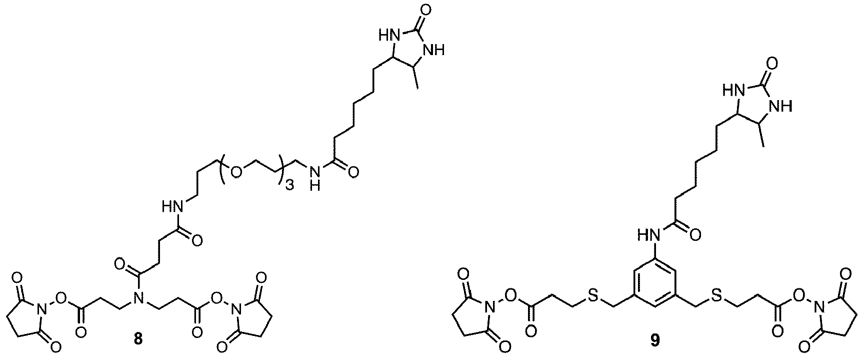

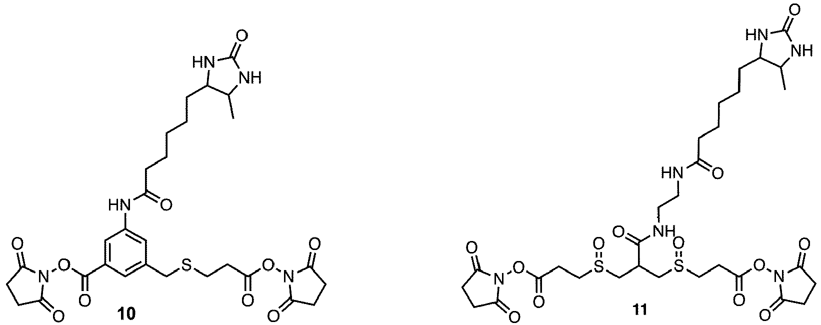

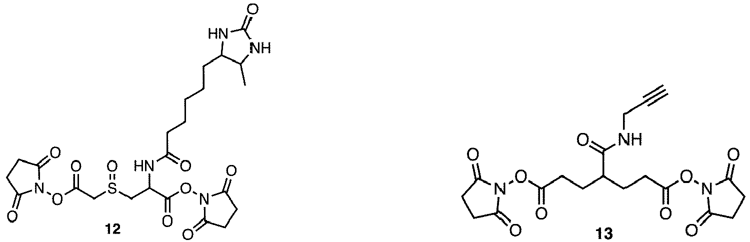

In one embodiment a preferred crosslinking agent is synthesized in a method comprising the following steps:
a) Reacting a compound of general formulae (la)

   R^{1a} - (CH₂)ₐ - XH - (CH₂)ₐ - R^{1a}

   wherein
   R^{1a} is an ester -COOR^{1b} with R^{1b} being a C₁-C₁₀ alkyl moiety, preferably a C₃-C₄ alkyl moiety, or another leaving group, preferably -Br, -I, -Cl, -OTs,-OMs, -OCOR, -ONO₂, -OPO(OH)₂ and
   X is selected from N, P, -CH-, aliphatic cyclic structures and aromatic cyclic structures;
   with at least one biotin derivative R² for obtaining a compound of general formulae (lb).

   R^{1a} - (CH₂)ₐ - XR²- (CH₂)ₐ - R^{1a}
b) adding at least one acid for hydrolysing agent to obtain a compound of general formulae (Ic)

   HOOC - (CH₂)ₐ - XR²- (CH₂)ₐ - COOH
c) Adding at least one compound of the general formulae (1d)

   R¹-Y or R¹-Nu

   Wherein
   Y is a leaving group like -Br, -I, -Cl, -OTs, -OMs, -OCOR, -ONO₂, -OPO(OH)₂ and
   Nu is a nucleophile like -NH₂, -OH, -SH.
   to obtain the compound of general formulae (I)

   R¹- (CH₂)ₐ - XR²- (CH₂)ₐ - R¹

In an embodiment X is selected from a group comprising -NH-, aniline derivatives, alkyl azides (preferably C₂-C₄), alkynes (preferably C₃-C₄), alkenes (preferably C₃-C₄), halide aryl (preferably C₅-C₆ such as C₆-C₁₄ aryl, C₅-C₁₄ heteroaryl), alkyl halides (preferably C₂-C₄), cycloalkyl halides (preferably C₅-C₆ such as C₃-C₁₀ cycloalkyl, C₃-C₁₀ cycloalkenyl, C₃-C₁₀ heterocycloalkyl, C₃-C₁₀ heterocycloalkenyl).

In a preferred embodiment of the present method the compound of the general formulae (1d) is selected from a group comprising ester, halogen, aldehyde, isothiocyanate, isocyanate, anhydride, epoxide, acetyl, glyoxal, triazine, hydrazine, disulfide, azide, ketone, phosphine, alkene, amine.

The compound of the general formulae (1d) may be selected from a group comprising N-Hydroxysuccinimide esters, imidoesters, isothiocyanates, isocyanates, acyl chlorides, sulfonyl chlorides, aryl sulfonyl fluorides, acyl azides, fluorophenyl esters, anhydrides, fluorobenzene, epoxides, alpha,beta-unsaturated aldehydes, 1,3-ketoaldehydes, 1,2,3-triazines, 1,2-cyclohexanedione, 2-methoxy-3-oxindoles, phenylglyoxal, a-keto-oximes, 2-fluoro-5-nitrotropolone, O-phthalaldehyde, maleimides, halo acetyls, pyridyl disulfides, aryl azides, diazirines, benzophenones, psoralens, phosphines, alkenes, cyclooctynes, tetrazines, hydrazines, alkoxyamines.

Further synthesis methods are illustrated in the Figures.

As mentioned above the present crosslinking agent is used for crosslinking mass spectrometry analysis. In particular, the present crosslinking agent is used for enriching at least parts of crosslinked peptides pairs.

Such a method for enriching at least parts of crosslinked peptides pairs comprises the steps of:
- Providing a mixture of at least one crosslinking agent as described above and at least one protein to be analysed,
- Digesting the protein by adding at least one proteolytic enzyme to obtain a peptide mixture of crosslinked peptides and linear peptides;
- Applying the peptide mixture to a streptadivin support, whereby the crosslinked peptides pairs will be bound to the streptadivin support and linear peptides are washed out; and
- Eluting the crosslinked peptides from the streptavidin support with an excess of biotin to obtain enriched crosslinked peptide pairs.

The crosslinking reaction is carried out in an amine-free aqueous buffer at or close to physiological pH (pH 7.2 - 7.8). Reaction time is typically between 30 minutes and 2 hours, carried out on ice or at room temperature. The crosslinking reaction is quenched by adding NH₄HCO₃ (ABC) at a concentration of 50 mM, with incubation for 15 minutes at room temperature.

The peptide mixture is applied to a streptadivin support, in particular streptavidin immobilized on Sepharose (highly crosslinked beaded agarose with high chemical stability, GE Healthcare).

Eluting may be done using a suitable buffer system such as PBS buffer system.

For the purpose of conducting the above enrichment process a kit may be provided, wherein the kit comprises
- at least one crosslinking agent as described above;
- Streptadivin attached to a solid support;
- optionally at least one proteolytic enzyme;
- a mobile phase / eluting agent.

The present invention provides a biotin-streptavidin system based enrichable crosslinker reagent which solves the problem of poor reversibility of biotin-streptavidin binding with the use of desthiobiotin, a biotin derivative with a weaker (but still substantial) binding affinity to streptavidin. Elution from solid-supported streptavidin is achieved via competitive elution, using an excess of biotin (in PBS buffer, pH 7.4), which acts as a competitive inhibitor for binding between streptavidin and desthiobiotin. The extremely mild elution conditions allowed by the competitive elution concept have few to no negative consequences for subsequent analyses, including by electrospray ionisation mass spectrometry. This approach may be described as crosslinker STAGEcl, for Stop And Go Extraction crosslinker, reflecting a continuation of heritage from the StageTips (Rappsilber, J., Ishihama, Y. & Mann, M. Stop and go extraction tips for matrix-assisted laser desorption/ionization, nanoelectrospray, and LC/MS sample pretreatment in proteomics. Anal. Chem. 75, 663-670 (2003)) widely used for peptide extraction in proteomics.

Thus, the invention provides the synthesis of an enrichable crosslinking reagent (STAGEcl), in a very simple three-step synthesis. STAGEcl comprises two protein reactive (NHS-ester) groups (that react predominantly with proximal lysine residues, but also with N-termini, tyrosine, threonine and serine residues) separated by a spacer, onto which a desthiobiotin moiety is grafted in the middle.

The invention provides also a means to do crosslinking mass spectrometry analysis on proteins inside living cells, organelles and cellular lysates, by enrichment of crosslinked and tagged peptides, where analysis is currently prohibited by the overabundance of linear background peptides.

The invention represents an enrichable, bioorthogonal, chemical reagent for bioconjugation, whereby two linked moieties (two analytes or one analyte and one probe) can be reversibly bound and released from a capture agent.

The invention represents further a trifunctional chemical reagent for bioconjugation, whereby one of the functionalities is an enrichable tag and two functionalities are reactive groups capable of forming covalent bonds.

Besides, the invention provides a means of reversible binding by competitive elution (using biotin contained in an extremely mild buffer (PBS, pH 7.4), of crosslinked analytes, including proteins and peptides. The invention comprises a biotin-alternative affinity reagent for use in chemical tags and crosslinking proteomics, which has greater water-solubility, is more chemically inert, less prone to aggregation, simpler and easier to elute from the solid-support.

The invention comprises also a chemistry for grafting a functionality containing a carboxylic acid derivative to a secondary amine on a crosslinker precursor backbone, via a tertiary amide, for the purposes of functionalized crosslinker synthesis.

The invention is explained in more detail with reference to examples and figures. It shows:
- Figure 1a-j: Synthesis of different crosslinkers according to the invention;
- Figure 2: GST crosslinking titration using a crosslinker according to the invention;
- Figures 3A-C: Crosslinking mass spectrometry analysis of Human Serum Albumin (HSA) crosslinked wit crosslinker 3 according to the invention;
- Figure 3D: Crosslinking and enrichment procedure, and
- Figure 3E: Enrichment of crosslinked peptide pairs.

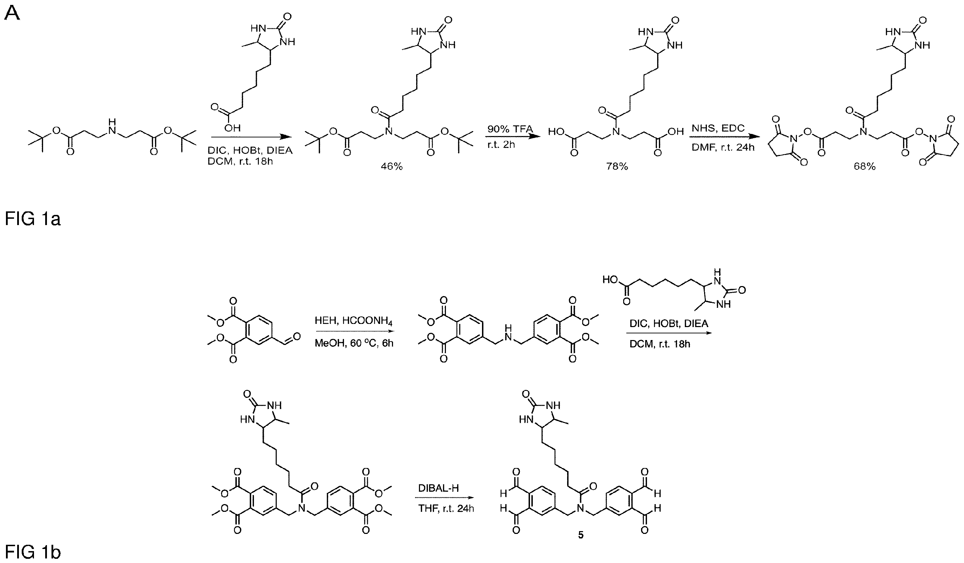

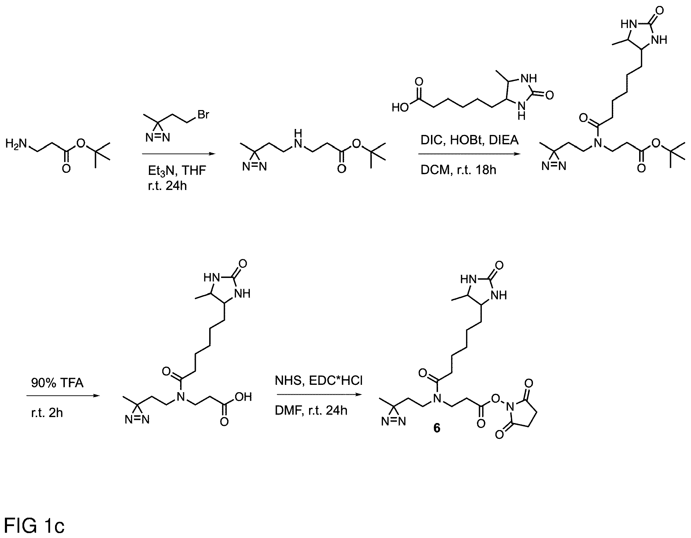

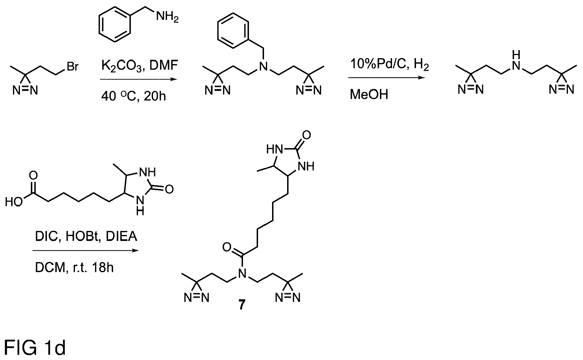

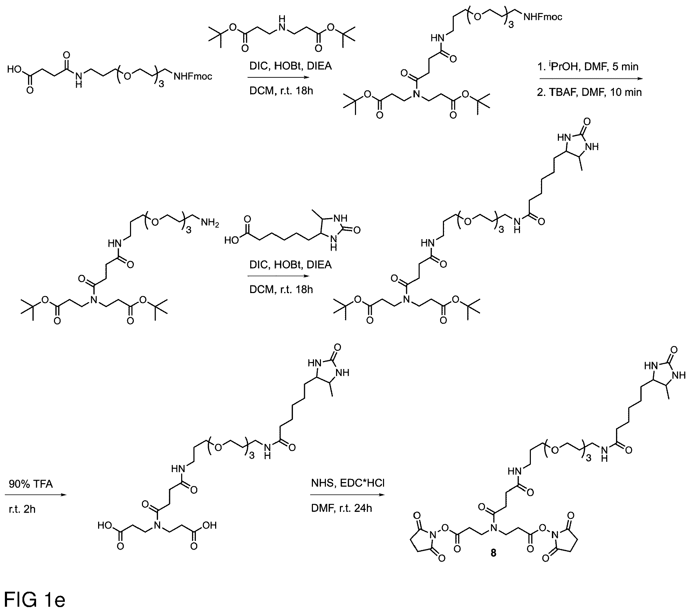

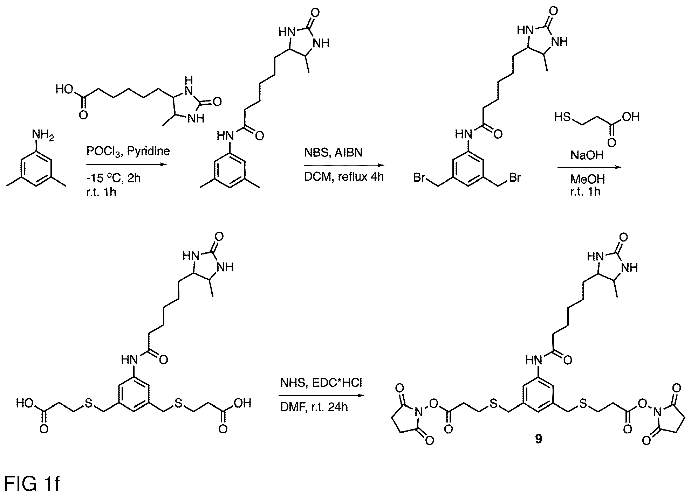

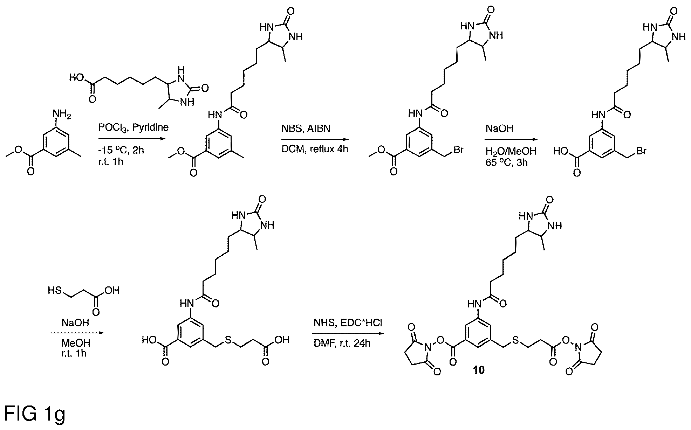

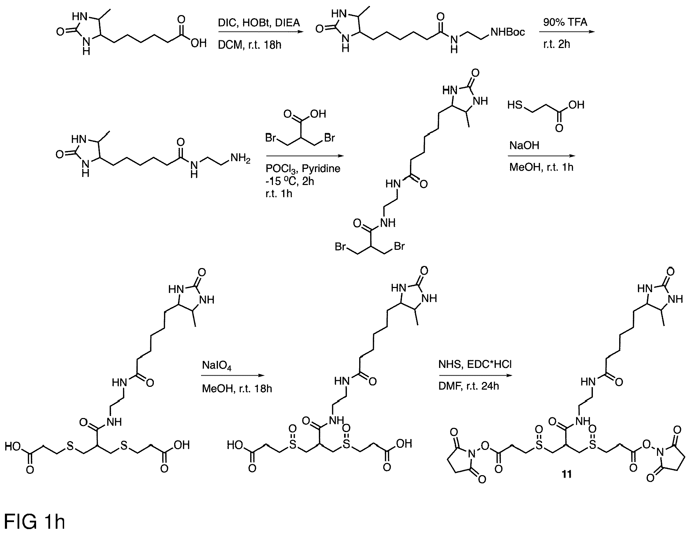

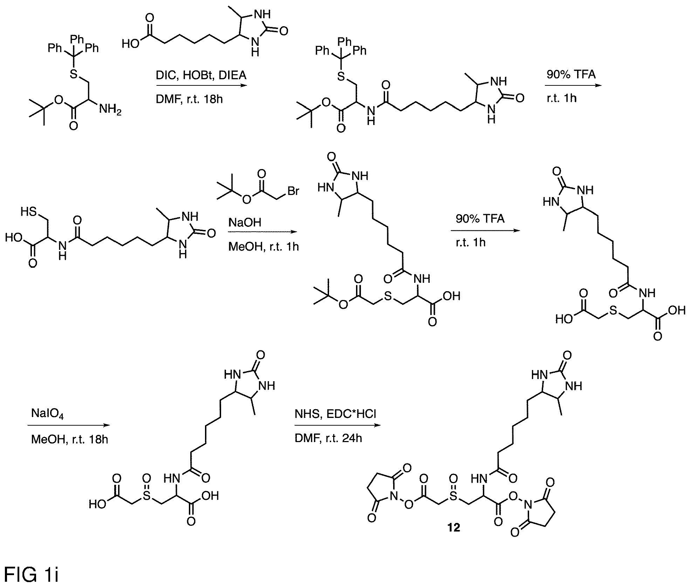

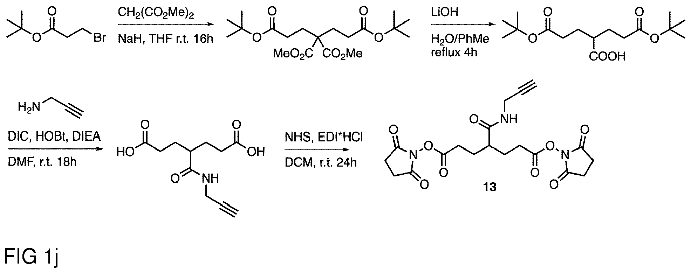

Figure 1A shows the three-step synthesis and final structure of one crosslinker 4 according to the invention (called STAGEcl). The finished crosslinking reagent constitutes NHS-ester protein reactive groups (reactive predominantly with the amino groups of lysine residues and protein N-termini, but also with the hydroxyl groups of serine, threonine and tyrosine), separated by a spacer, which has a desthiobiotin moiety grafted in the middle, via a tertiary amide.

### Synthesis protocol of Crosslinker 4

### General Methods

Chemicals and solvents were purchased from Fisher Scientific, Sigma-Aldrich, VWR International Ltd or TCI UK Ltd. NMR spectra were recorded at ambient temperature on a 500 MHz Bruker Avance III spectrometer. Chemical shifts are reported in parts per million (ppm) relative to the solvent peak. Rf values were determined on Merck TLC Silica gel 60 F254 plates under a 254 nm UV source. Purification was carried out by flash chromatography using commercially available Silica 60 Å, particle size 40-63 micron under positive pressure. Compounds purity was >95% pure, as measured by LC-MS using an UV-Vis 254 nm detector and Bruker ESI Micro-Tof mass spectrometer. Method was eluent A: water and trifluoroacetic acid (0.4%); eluent B: acetonitrile; A/B = 95:5 to 20:80 in 6 min, isocratic 1 min, 20:80 to 95:5 in 0.1 min, and isocratic 2 min.

### Synthesis of di-tert-butyl 3,3'-((6-(5-methyl-2-oxoimidazolidin-4-yl)hexanoyl)azanediyl) dipropionate.

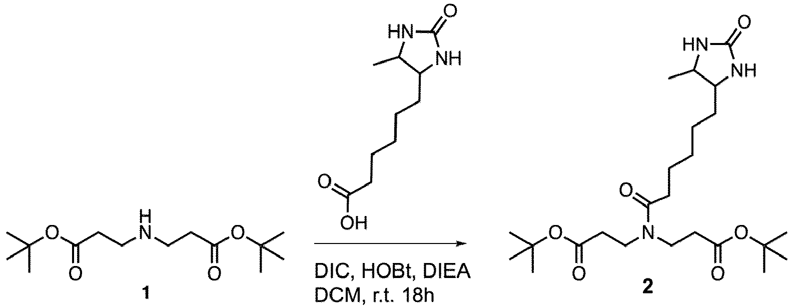

d-Desthiobiotin (500 mg, 2.33 mmol) and 1-hydroxybenzotriazole hydrate (HOBt) (530 mg, 3.50 mmol) were dissolved in dry DCM (25 mL) under a nitrogen atmosphere. N,N-Diisopropylethylamine (DIEA) (1.1 mL, 6.99 mmol) was added dropwise and a cloudy solution was formed. Then, N,N'-diisopropylcarbodiimide (DIC) (545 L, 3.50 mmol) was added dropwise and the mixture was stirred for 10 mins. A solution of di-*tert*-butyl 3,3'-iminodipropionate (**1,** 650 L, 2.33 mmol) in dry DCM (1 mL) was then added under a nitrogen atmosphere. After the reaction was stirred overnight at room temperature, the mixture was concentrated under reduced pressure. The crude residue was dissolved in DCM and washed with water (3 x 20 mL), HCI 5% 2N (3 x 20 mL) and brine (20 mL). The organic layer was dried using anhydrous MgSO₄ and the solvent was evaporated *in vacuo.* The resulting residue was purified by flash chromatography (2.5% MeOH in DCM) to yield **2** as a pale oil (496 mg, 46% yield). Rf = 0.44 (5% MeOH in DCM). ¹H NMR (500 MHz, DMSO) δ ¹H NMR (500 MHz, DMSO) δ 6.30 (s, 1H), 6.11 (s, 1H), 3.67 - 3.58 (m, 1H), 3.53 - 3.49 (m, 2H), 3.40 (t, *J* = 7.2 Hz, 2H), 2.49 (d, *J* = 7.1 Hz, 2H), 2.40 (t, *J* = 7.2 Hz, 2H), 2.29 (t, *J* = 7.4 Hz, 2H), 1.47 (p, *J* = 7.4 Hz, 2H), 1.40 (d, *J* = 2.9 Hz, 18H), 1.38 - 1.13 (m, 6H), 1.01 (d, *J* = 6.5 Hz, 2H), 0.96 (d, *J* = 6.4 Hz, 2H). ¹³C NMR (126 MHz, DMSO) δ 172.40, 171.23, 170.96, 163.25, 80.70, 80.33, 55.45, 50.68, 49.07, 43.91, 41.81, 41.14, 35.13, 33.94, 32.49, 29.99, 29.21, 28.18, 28.16, 26.19, 25.17, 23.77, 15.96. HRMS (ESI) *m*/*z* [M + H]⁺ calcd for C₂₄H₄₄N₃O₆, 470.32246; found 470.32270.

### Synthesis of 3,3'-((6-(5-methyl-2-oxoimidazolidin-4-yl)hexanoyl)azanediyl)dipropionic acid.

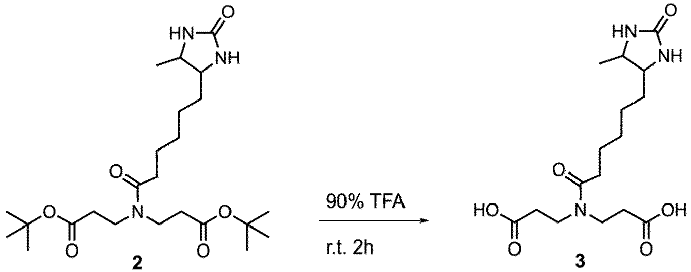

Compound **2** (496 mg, 1.05 mmol) was treated with a trifluoroacetic acid (TFA) solution (90% in water) for 2 h at room temperature. The mixture was then concentrated under reduced pressure. The crude was purified by SPE Cartridges Chromabond C18, 6mL/1000mg (30% MeOH in H₂O) to yield **3** as a pale oil (292 mg, 78% yield).¹H NMR (500 MHz, DMSO) δ 6.29 (s, 1H), 6.10 (s, 1H), 3.60 (ddt, *J* = 9.6, 6.3, 3.5 Hz, 1H), 3.52 (t, *J* = 7.4 Hz, 2H), 3.40 (t, *J* = 7.4 Hz, 2H), 2.53 - 2.51 (m, 1H), 2.47 (s, 1H), 2.40 (t, *J* = 7.3 Hz, 2H), 2.29 (t, *J* = 7.4 Hz, 2H), 1.47 (p, *J* = 7.4 Hz, 2H), 1.37 - 1.16 (m, 6H), 1.00 (d, *J* = 6.5 Hz, 1H), 0.96 (d, *J* = 6.4 Hz, 3H). ¹³C NMR (126 MHz, DMSO) δ 182.56, 182.22, 181.44, 172.32, 64.48, 59.71, 53.11, 51.00, 43.21, 41.97, 41.41, 39.00, 38.22, 35.19, 34.18, 32.78, 24.98. HRMS (ESI) *m*/*z* [M + H]⁺ calcd for C₁₆H₂₈N₃O₆, 358.19726; found 358.19680.

### NHS activation of 3,3'-((6-(5-methyl-2-oxoimidazolidin-4-yl)hexanoyl)azanediyl) dipropionic acid.

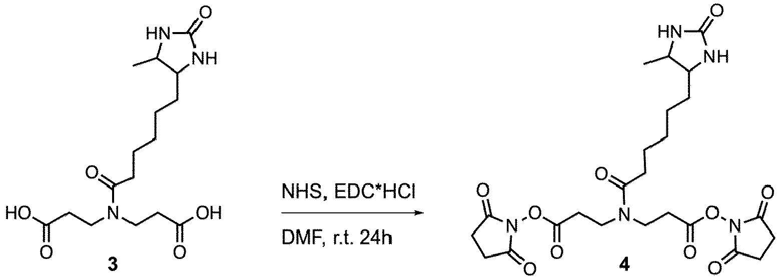

Compound **3** (292 mg, 0.82 mmol) and N-hydroxysuccinimide (190 mg, 1.64 mmol) were dissolved in dry DMF (5 mL) under a nitrogen atmosphere, followed by addition of 1-ethyl-3-(3-dimethylaminopropyl) carbodiimide hydrochloride (EDC·HCl) (289 mg, 1.64 mmol) dissolved in dry DMF (1 mL). The mixture was stirred for 24 h at room temperature. The solvent was evaporated *in vacuo* and the solid was dissolved in EtOAc (10 mL), which was then washed with sat. NaHCO₃ (2 x 10 mL), 10% citric acid (2 x 10 mL) and brine (10 mL). The organic layer was dried using anhydrous MgSO₄ and the solvent was evaporated *in vacuo* to yield **4** as a colourless oil (303 mg, 68%). Rf = 0.50 (10% MeOH in DCM). ¹H NMR (500 MHz, DMSO) δ 6.28 (s, 1H), 6.10 (s, 1H), 3.70 (t, *J* = 6.9 Hz, 2H), 3.60 - 3.56 (m, 2H), 3.51 - 3.47 (m, 1H), 3.07 (t, *J* = 6.9 Hz, 2H), 2.93 (t, *J* = 7.2 Hz, 2H), 2.82 (s, 8H), 2.35 (t, *J* = 7.5 Hz, 2H), 1.49 (p, *J* = 7.5 Hz, 2H), 1.41 - 1.14 (m, 6H), 1.01 (d, *J* = 6.5 Hz, 1H), 0.96 (d, *J* = 6.4 Hz, 3H). ¹³C NMR (126 MHz, DMSO) δ 172.94, 170.61, 170.57, 168.01, 167.88, 163.26, 162.77, 55.46, 50.69, 43.56, 41.54, 41.14, 36.25, 32.47, 31.25, 30.54, 29.99, 29.39, 29.17, 26.22, 25.92, 25.01, 23.77, 15.97. HRMS (ESI) *m*/*z* [M + H]⁺ calcd for C₂₄H₃₄N₅O₁₀, 552.23002; found 552.22970.

Figure 1b-j show the synthesis and final structure of further crosslinkers 5-13 according to the invention.

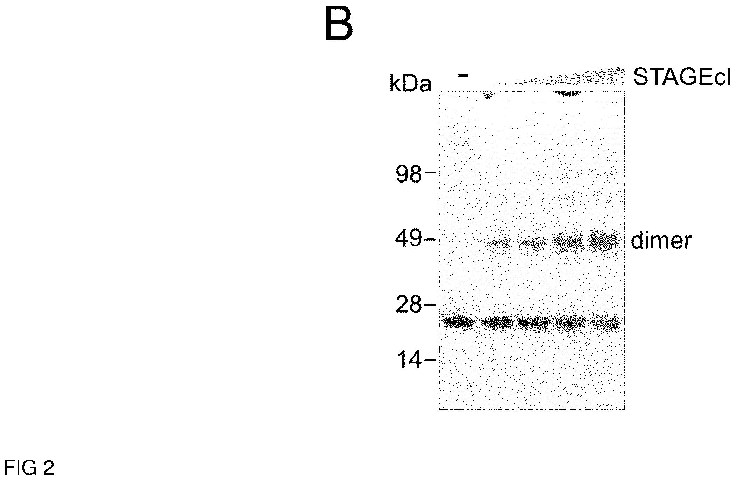

Figure 2 shows the SDS-PAGE resulting from crosslinking of Glutathione S-Transferase (GST) dimer with increasing amounts of STAGEcl (crosslinker 4). GST runs on SDS-PAGE as a 25 kDa monomer under denaturing conditions. Crosslinked dimer can be observed as a result of crosslinking and increasing the ratio crosslinker:protein results in increased formation of crosslinked GST dimer.

GST in 2 µg aliquots was crosslinked (0.33 µg/µL) in crosslinking buffer (20 mM HEPES, 20 mM NaCl, 5 mM MgCl2, pH 7.8) using different crosslinker:protein ratios (w/w): 0.15:1, 0.44:1, 1.3:1 and 4:1. Crosslinking was carried out for 1 h at room temperature, after which the reaction was quenched with 50 mM ABC, with incubation for 20 mins at room temperature. Crosslinked protein samples were separated by SDS-PAGE on a 1 mm thick NuPAGE 4-12% Bis-Tris SDS-PAGE gel, using MES running buffer and Coomassie blue stain.

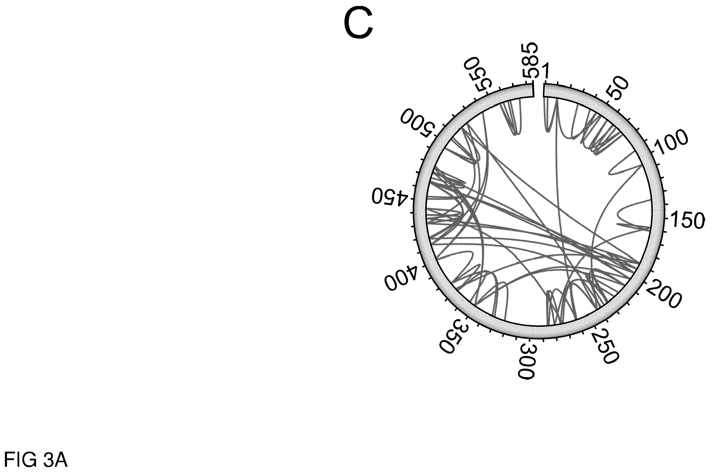

Figure 3A shows a crosslinked network resulting from STAGEcl crosslinking mass spectrometry analysis of human serum albumin (HSA) with crosslinker 4. The outer circular line represents the protein sequence. Links shown are at a 5% FDR level.

HSA (200 µg, 0.5 µg/µL) in crosslinking buffer (20 mM HEPES, 20 mM NaCl, 5 mM MgCl2, pH 7.8) was crosslinked using STAGEcl (crosslinker 4) in a crosslinker:protein (w/w) ratio of 1.64:1, for 1 h at room temperature. The reaction was then quenched with 50 mM ABC. Crosslinked protein was separated by SDS-PAGE on a 1.5 mm thick NuPAGE 4-12% Bis-Tris SDS-PAGE gel using MES running buffer and Coomassie blue stain.

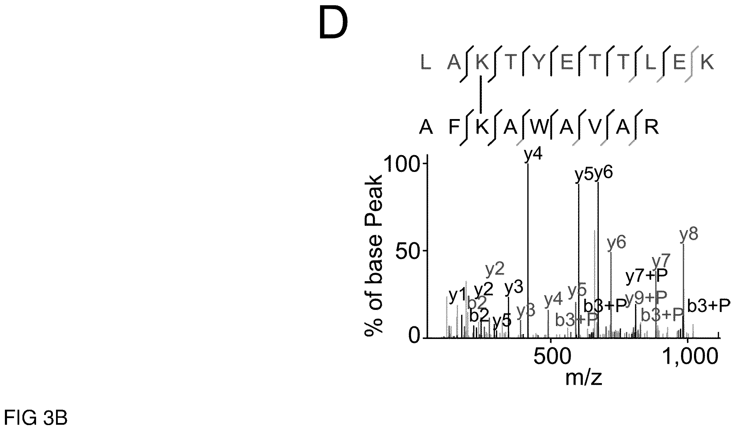

Figure 3B shows a high-resolution fragmentation spectrum of a matched STAGEcl (crosslinker 4) crosslinked peptide pair. The sequences of the matched crosslinked peptides, Peptide 1 (red, upper line) and Peptide 2 (black, lower line), are given by the single letter amino acid code. The crosslinking site (K-K) is represented by the solid black line between residues, connecting both peptide sequences. Crosslinked peptide pairs are matched by database search according to precursor m/z (obtained by an MS1 (survey) scan), and the combination of m/z fragments identified in the mass spectrometer following MS2 fragmentation. The fragment ions detected for each peptide are indicated by vertical lines between amino acid residues. Fragment ions belong to either a γ-series (indicated by a top tick on the vertical line) or b-series (indicated by a bottom tick on the vertical line). The presented fragmentation spectrum shows the m/z of b- and y-ions identified according to the matched peptide sequence.

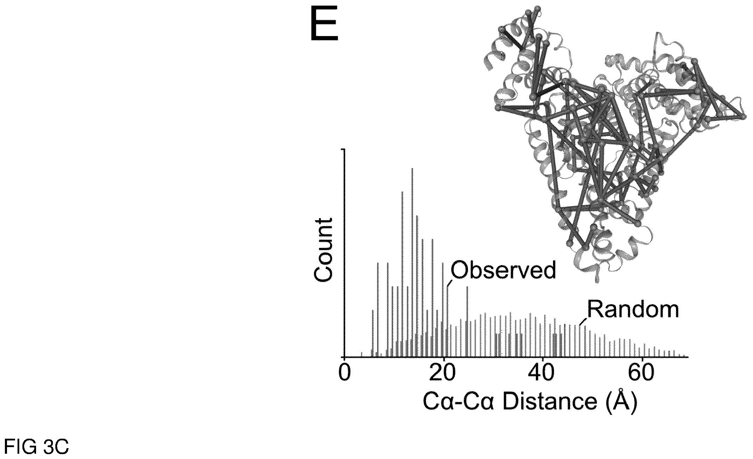

Figure 3C shows links (indicated by red lines) between residue pairs (5% FDR) fitted to the solved x-ray crystal structure (PDB 1A06, cartoon representation in grey). The histogram shows the observed Ca-Ca distance distribution (in angstroms) for observed linked residue pairs (red), against the random distance distribution (grey) where all crosslinked distances are considered. STAGEcl has an estimated upper distance constraint for crosslinked K-K residues of 27 Å. The majority of crosslinked residues have Ca-Ca distance <20 Å, falling well within the expected distance distribution. The crosslinked residue pairs that exceed this estimated distance fall within the range of expected false positive matches according to the FDR estimation.

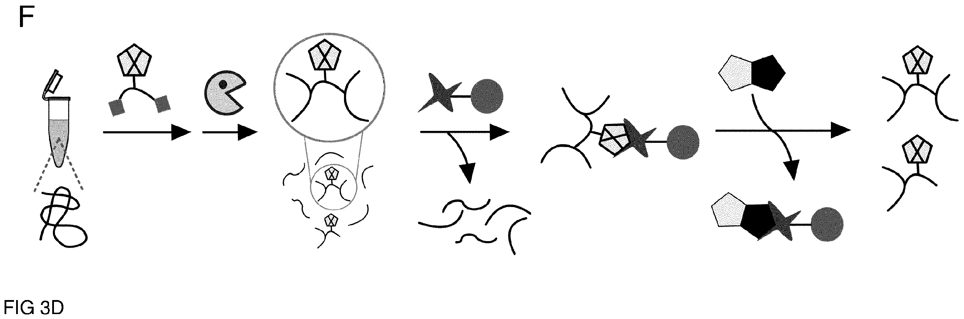

Figure 3D shows a scheme of the protein crosslinking, digestion and enrichment process. Following the crosslinking reaction, the crosslinked proteins can be proteolytically digested, the resulting peptide mixture applied to solid-supported streptavidin in a PBS buffer, and crosslinked peptides bound to the solid-support with high specificity and efficiency. Linear modified peptides are then washed from the solid-support, and competitive elution with an excess of biotin is achieved, also in PBS buffer, pH 7.4. The resulting enriched crosslinked peptide pairs can then be either analysed directly by mass spectrometry, or subjected to further chromatographic fractionation, including (but not limited to) SCX, SEC and hSAX.

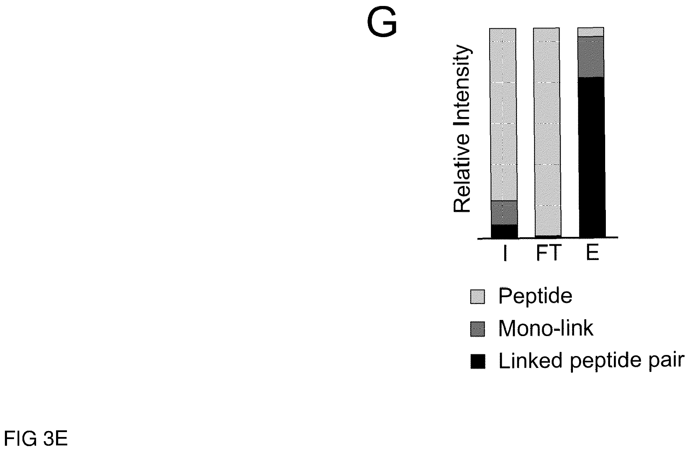

Figure 3E shows the level of enrichment following the enrichment procedure on a STAGEcl (crosslinker 4) crosslinked *E.coli* lysate digest. The three columns show the relative intensity of matched MS1 precursors for non-crosslinker and crosslinker modified peptides (comprised of mono-linked peptides and crosslinked peptide pairs) at different stages of enrichment: (1) input to beads (without enrichment), (FT) the flow-through from beads during enrichment and (E) eluate from beads after enrichment. The bars representing the input (1) and flow-through (FT) were the result of the respective samples following an additional SEC fractionation. The bar representing the eluate (E) was the result of enrichment (affinity enrichment-SCX-SEC).

### Cell production

A single clone of *E.coli* K12 strain (BW25113, DSMZ, Germany) grown on agar plates was selected for inoculation of lysogeny broth (LB)-media. Fermentation was initiated in a Biostat A Plus Bioreactor (Sartorius, Göttingen, Germany) using a preculture aliquot in LB medium, with 0.5% (w/v) glucose, at 37 °C. Growth was monitored frequently by taking optical density measurements at 600 nm. Fermentation was stopped at an optical density of 10 by rapidly cooling the culture in stirred ice water followed by biomass harvesting by centrifugation at 5000 g, 4 °C for 15 mins. Cell pellets were snap-frozen in liquid nitrogen and stored at -80 °C.

### Cell lysis

Cell pellets were resuspended in ice-cold lysis buffer (50 mM HEPES, pH 7.4 at RT, 50 mM KCI, 50 mM NaCl, 1.5 mM MgCl₂, 5% (v/v) glycerol, 1 mM dithiothreitol (DTT), a spatula tip of chicken egg white lysozyme (Sigma-Aldrich, St. Louis, MO, USA) and complete EDTA-free protease inhibitor cocktail (Roche, Basel, Switzerland)). Cells were lysed by sonication on ice at 30% amplitude, 30 s on/off for 10 cycles (total time 5 mins), using a Branson Digital Sonifier.

After sonication, 125 units of Benzonase (Merck, Darmstadt, Germany) were added. Liquid was collected in a centrifuge tube (upper foaming with DNA proteins was discarded) and sample was clarified by centrifugation at 15,500 rpm for 30 mins at 4 °C. DTT was added to 2 mM. The cleared lysate was then subjected to ultracentrifugation using a 70 Ti fixed-angle rotor for 1h at 106,000 g at 4 °C, after which the supernatant was concentrated 10x using ultrafiltration with Amicon spin filters (15 kDa molecular weight cut-off) to achieve a total protein concentration of 10 mg/mL, determined by microBCA protein assay (Thermo Fisher Scientific, Waltham, MA, USA).

### E.coli lysate crosslinking

Cell lysate was diluted to 1 mg/mL protein concentration with crosslinking buffer (50 mM HEPES, 50 mM NaCl, 50 mM KCI, 1.5 mM MgCl2, 5% glycerol, pH 7.4). Cell lysate was then crosslinked with 1 mM STAGEcl (crosslinker 4) for 1 h at room temperature. After this time the crosslinking reaction was quenched with 50 mM ABC, with incubation for 30 mins on ice. Crosslinked proteins were acetone-precipitated overnight at -20 °C. Protein was solubilised in 6 M urea, 2 M thiourea, 100 mM ABC. Protein sample was then subjected to proteolysis with LysC added at a 1:100 (m/m) ratio followed by incubation for 4 h at 37 °C. After 1:5 dilution with 100 mM ABC, trypsin was added at a ratio of 1:25 (m/m) and the digestion allowed to continue for 16 h at 37 °C, after which digestion was stopped with the addition of TFA to 1% (v/v). Digests were desalted using SPE cartridges according to the manufacturer's instruction and eluates dried, aliquoted and stored at -20 °C until further use.

## Claims

1. Crosslinking reagent for bioconjugation for use in crosslinking proteomics, in particular crosslinking mass spectrometry analysis, of the general structure of general formula (I)
R¹-(CH₂)ₐ - (Y-CH₂)ₙ-XR²- (CH₂-Y)ₙ - (CH₂)ₐ - R¹
Wherein
- R¹ is selected from a functional group comprising ester, halogen, aldehyde, isothiocyanate, isocyanate, anhydride, epoxide, acetyl, glyoxal, triazine, hydrazine, disulfide, azide, ketone, phosphine, alkene, amine, N-heterocycle,
- a = 1-10, preferably 2-5, more preferably 2-3;
- Y is selected from -O-, -S-, -S-S-, -S(=O)-, -¹³CH₂-, -CD₂-, -¹⁸O-m, in particular -O-, -S-, -S-S-;
- n = 0-4, preferably 0, 1 or 2,- X is selected from N, P, -CH-, aliphatic cyclic structures and aromatic cyclic structures, preferably N;
- R² comprises a moiety, in particular a biotin derivative, which has a lower affinity to avidin or streptavidin than biotin.

2. Crosslinking reagent according to claim 1, **characterised in that** R¹ is selected from a group comprising N-hydroxysuccinimide esters, imidoesters, isothiocyanates, isocyanates, acyl chlorides, sulfonyl chlorides, aryl sulfonyl fluorides, acyl azides, fluorophenyl esters, anhydrides, fluorobenzene, epoxides, alpha,beta-unsaturated aldehydes, 1,3-ketoaldehydes, 1,2,3-triazines, 1,2-cyclohexanedione, 2-methoxy-3-oxindoles, phenylglyoxal, a-keto-oximes, 2-fluoro-5-nitrotropolone, O-phthalaldehyde, maleimides, halo acetyls, pyridyl disulfides, aryl azides, diazirines, benzophenones, psoralens, phosphines, alkenes, cyclooctynes, tetrazines, hydrazines, alkoxyamines.

3. Crosslinking reagent according to claim 1, **characterised in that** R¹ is a succinimide ester (NHS), phthalic-di-aldehyde, diazirine.

4. Crosslinking reagent according to any of the preceding claims, **characterised in that** R² comprises a biotin derivative wherein the cyclic moiety of biotin different from that of biotin such as desthiobiotin, 2-iminobiotin, 3,4-diaminobiotin or chemically modified derivatives.

5. Crosslinking agent according to one of the preceding claims, **characterised in that** R² comprises desthiobiotin.

6. Crosslinking agent according to one of the preceding claims, **characterised in that** the crosslinking agent is of an asymmetrical structure (II) 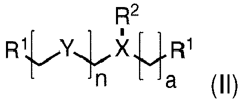 or a symmetrical structure (III) 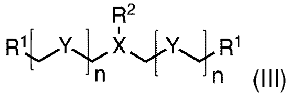 wherein
- a = 1-10, preferably 2-5, more preferably 2-3.
- n = 1-4, preferably 1-2, more preferably 1.
- Y is selected from -O-, -S-, -S-S-, -S(=O)-, -¹³CH₂-, -CD₂-, -¹⁸O-.

7. Crosslinking agent according to one of the preceding claims, **characterised in that** the crosslinking agent is of

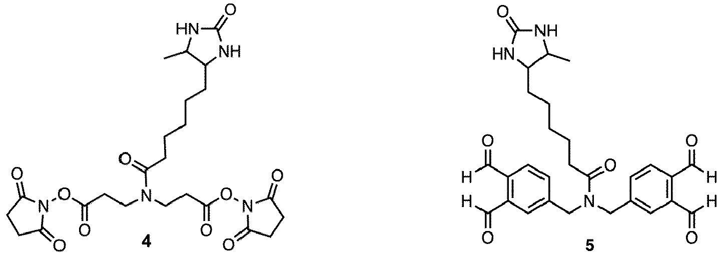

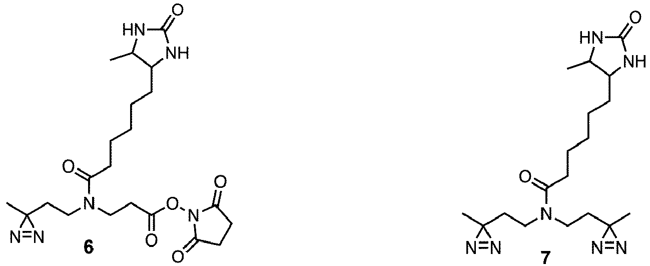

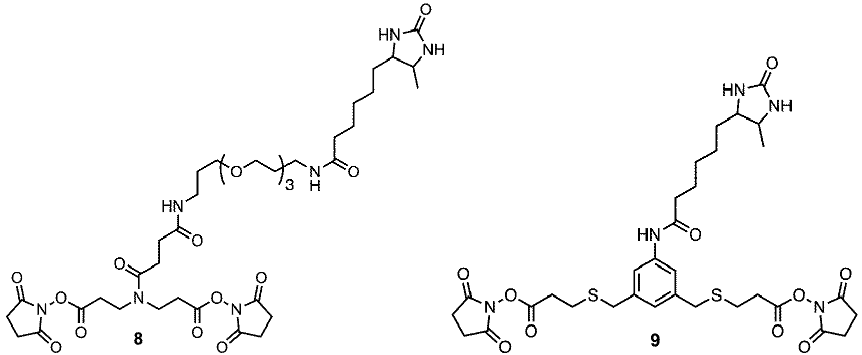

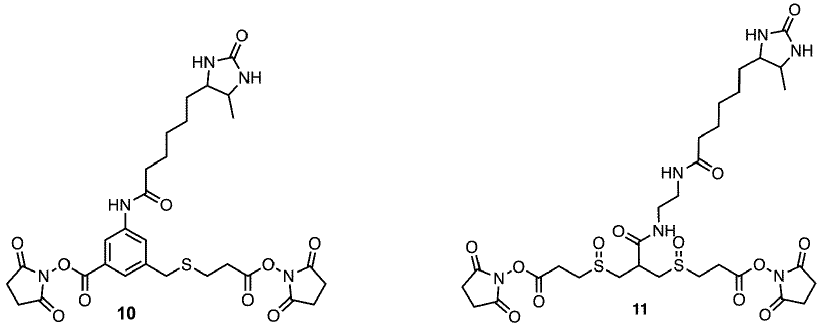

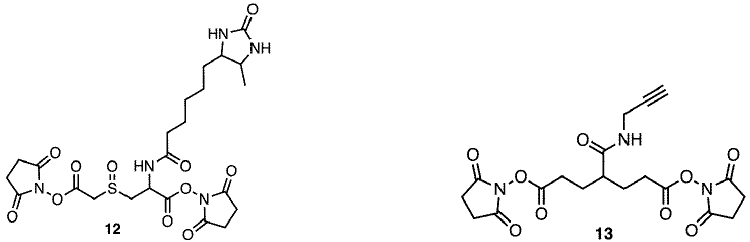

8. Use of a crosslinking agent according to one of the claims 1-7 for crosslinking mass spectrometry analysis.

9. Method of enriching at least parts of crosslinked peptides pairs comprising the steps of:
- Providing a mixture of at least one crosslinking agent according to one of the claims 1-7 and at least one protein to be analysed.
- Digesting the protein by adding at least one proteolytic enzyme to obtain a peptide mixture of crosslinked peptides and linear peptides;
- Applying the peptide mixture to a streptavidin support whereby the crosslinked peptides pairs will be bound to the streptavidin support and linear peptides are washed out; and
- Eluting the crosslinked peptides from the streptavidin support with an excess of biotin to obtain enriched crosslinked peptide pairs

10. Kit comprising
- at least one crosslinking agent according to one of the claims 1-7;
- Streptavidin attached to a solid support;
- optionally at least one proteolytic enzyme;
- a mobile phase / eluting agent.
